# EUROPEAN PATENT APPLICATION

(11) **EP 0 605 161 A2**
(43) Date of publication of application: **06.07.1994**
(21) Application number: 93310276.6
(22) Date of filing: 20.12.1993
(51) Int. Cl.: A61K 31/71

(54) **Lowering rhematoid factor levels**

(30) Priority: 29.12.1992 US 998353
(71) Applicant: AMERICAN HOME PRODUCTS CORPORATION, Madison, New Jersey 07940-0874 (US)
(72) Inventor: Scarola, Joseph Alexander, Montgomery, Pennsylvania 19446 (US)
(74) Representative: Wileman, David Francis, Dr.

(57) **Abstract**

This invention provides a method of lowering rheumatoid factor levels in a mammal by administering rapamycin.

## Description

This invention relates to a method of lowering rheumatoid factor levels, in particular to a new use of rapamycin.

Rheumatoid arthritis (RA) is a chronic, systemic inflammatory disease of unknown etiology that affects approximately 0.5 to 3.8% of women and 0.15 to 1.3% of men, with the most frequent age of onset between ages 25-50. The principal clinical manifestation is a progressive deforming arthritis of multiple joints. RA typically begins in the small joints of the hands and feet and progresses in a centripetal and symmetric fashion. Thus, the wrists, elbows, ankles, knees, and spine can also be affected. Patients generally experience stiffness and joint pain that are worse in the morning and improve throughout the day. Articular inflammation, including swelling, warmth, erythema, and tenderness on palpation are observed in beginning in the early stages of the disease. In the advanced stages of the disease, muscle spasm, limitation of motion, muscle contractions, and ankylosis with permanent joint deformity are observed. [Basic and Clinical Immunology, D. P. Stites, ed., 7th ed., Appleton and Lange, 443 (1991)].

In the early stages of RA, there is a non-specific acute edematous inflammation marked by infiltration of the synovia by neutrophils, lymphocytes, and plasma cells. As the disease progresses, the synovial lining becomes hypertrophied and multilayered with the subsynovial tissue also undergoing a vascularized hyperplasia. The result of this process is that the synovial lining becomes replaced with a highly vascularized mass of inflammatory tissue infiltrated primarily by lymphocytes, macrophages, and plasma cells, known as the pannus. As the disease progresses, the pannus and the inflammatory reaction erode the underlying articular cartilage, the joint capsule, and supporting ligaments causing weakened joint support and dysfunction. In the later stages of the disease, the total articular surface is eroded and the joint space obliterated. [Pathologic Basis of Disease, C. Robbins, ed., 3rd ed., Saunders, 1351 (1984)]. In addition to joint destruction, extra-articular manifestations such as rheumatoid nodules, which appear on the skin of approximately 20-25% of RA patients, may complicate the disorder.

While the etiology of RA is unknown, it is generally accepted that the synovitis caused by RA is autoimmune mediated. In most patients with RA, antibodies against the Fc fragment of IgG, called rheumatoid factors (RF), are detected in the joints and circulation. The autoantibodies are of the IgM, IgG, and IgA classes and form heterogeneous complexes with autologous IgG. IgE rheumatoid factor also has been reported. The formation of the RF-IgG complexes binds complement causing a self-perpetuating inflammatory response leading to the joint destruction described above. Levels of RF can be measured using the Waaler-Rose and latex fixation techniques which mainly detect IgM antibodies and with ELISA techniques that are capable of measuring all the RF isotypes. It is generally accepted that the serum titer of RF is roughly correlated with the severity of the RA, and that decreasing the RF levels by preventing the formation of RF is indicative of successful treatment of RA. [Basic and Clinical Immunology, D. P. Stites, ed., 7th ed., Appleton and Lange, 443 (1991); Pathologic Basis of Disease, C. Robbins, ed., 3rd ed., Saunders, 1351 (1984); Williams, R., J. Rheumatol. 19 (Suppl. 32): 42 (1992); Mannik, M. , J. Rheumatol. 19 (Suppl. 32): 46 (1992); van Zeben, D., Ann. Rheum. Dis. 51: 1029 (1992).

Approximately 75% of RA patients show improvement with conservative treatment during the first year after being diagnosed with RA; however in 5-10% of RA patients, all treatments are ineffective and these patients are eventually disabled. Conservative treatment is generally attempted with first line drugs such as aspirin or other non-steroidal antiinflammatory drugs. For patients not benefited by conservative therapy, second line therapies include treatment with gold compounds, D-penicillamine, hydroxychloroquine, corticosteroids, and immunosuppressive agents, such as cyclophosphamide, methotrexate, and azathioprine. These treatments, however, are limited by the high incidences side effects associated with their use. [The Merck Manual, 15th ed. 1239 (1987); Madhok, R., J. Rheumatol. 18: 1485 (1991)].

Several studies have been reported for the use of cyclosporin A (CsA), an immunosuppressive agent useful in preventing transplant rejection, in the treatment of rheumatoid arthritis. The results have been mixed: CsA failed to lower levels of rheumatoid factor [Aust. N.Z. J. Med. 21: 844 (1991); Scand. J. Rheumatol. Suppl. 76: 265 (1988)]; CsA lowered rheumatoid factor levels in a single patient with Felty's Syndrome [Arthritis Rheum. 34: 253 (1991)]. CsA was shown to produce clinically significant improvements in several patients with RA [Madhok, R., J. Rheumatol. 18: 1485 (1991); Yocum, D., Ann. Internal Med. 109: 863 (1988)]. RF levels were not measured in this study.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal, J. Antibiot. 28, 727 (1975); H. A. Baker, J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies. Rapamycin has been shown to produce dose related results in both developing and established adjuvant arthritis rat models [Carlson, R., FASEB 4: A1021 (1990)] and inhibited disease progression in a collagen induced arthritis murine model of arthritis [Adams, L., FASEB 4: A358 (1990)].

The immunosuppressive effects of rapamycin on T-lymphocytes have been disclosed in FASEB 3, 3411 (1989). Rapamycin was also shown to inhibit pokeweed mitogen induced IgG, IgM, and IgA production by peripheral blood mononuclear cells, and inhibited IgG and IgM production in tonsillar B-cell stmulated with IL-2 and Staphylococcus aureus Cowan I [Luo, H., Transplantation Proceedings 23: 2236 (1991); Luo, H., Transplantation 53: 1071 (1992)]. The ability of rapamycin to prolong survival time in histoincompatible rodents was disclosed by R. Morris [Med Sci. Res. 17: 877 (1989)] and to prevent transplantation rejection was disclosed in U.S. Patent 5,100,899.

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], and smooth muscle cell proliferation and intimal thickening following vascular injury [Morris, R. J. Heart Lung Transplant 11 (pt. 2): 197 (1992)].

This invention provides use of rapamycin in the preparation of a medicament for lowering rheumatoid factor levels in a mammal.

This invention also provides a method of lowering rheumatoid factor levels in a mammal in need thereof by administering a rheumatoid factor lowering effective amount of rapamycin to said mammal orally, parenterally, intranasally, intrabronchially, transdermally, or rectally.

Rapamycin is useful in lowering RF levels in those mammals with suspected or diagnosed RA who present with elevated RF levels. RA is readily diagnosable by physical examination, measurement of levels of rheumatoid factors, measurement of erythrocyte sedimentation rate (ESR), and radiologic evaluation. Standard diagnostic criteria for RA have been established. [Primer on Rheumatic Diseases, Rodnan, ed., 8th ed, (1983)]. The methods available for measuring RF levels were described above. This invention is particularly useful to those patients who are refractory to current forms of first line therapy because of the ability of rapamycin to prevent immunoglobulin formation.

When rapamycin is administered to lower levels of rheumatoid factor, it can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Rapamycin may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. rapamycin may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

Rapamycin may be administered topically as a solution, cream, or lotion by formulation with pharmaceutically acceptable vehicles containing 0.1 - 5 percent, preferably 2%, of active compound.

The dosage requirements vary with the particular compositions employed, the route of administration, the severity of the symptoms presented and the particular subject being treated. Projected daily intravenous dosages of rapamycin would be 0.001 - 25 mg/kg, preferably between 0.005 - 5 mg/kg, and more preferably between 0.01 - 0.5 mg/kg. Projected daily oral dosages of rapamycin would be 0.005 - 75 mg/kg, preferably between 0.01 - 50 mg/kg, and more preferably between 0.05 - 10 mg/kg.

Treatment will generally be initiated with small dosages less than the optimum dose of the compound. Thereafter the dosage is increased until the optimum effect under the circumstances is reached; precise dosages for oral, parenteral, intranasal, intrabronchial, transdermal, or rectal administration will be determined by the administering physician based on experience with the individual subject treated.

## Claims

1. Use of rapamycin in the preparation of a medicament for lowering rheumatoid factor levels in a mammal.

2. Use according to Claim 1 in which the medicament is adapted for administration orally, parenterally, intranasally, intrabronchially, transdermally, or rectally.

3. Use according to claim 1 wherein the mammal to be treated suffers from rheumatoid arthritis.

4. Use according to claim 3 wherein the mammal to be treated is refractory to treatment with aspirin or other non-steroidal antiinflammatory drug.

5. Use according to any one of claims 1 to 4 wherein the medicament is administered intravenously.

6. Use according to claim 5 wherein the rapamycin is administered in a dose range from 0.001 to 25 mg/kg per day.

7. Use according to claim 5 wherein the rapamycin is administered in a dose range from 0.005 to 5 mg/kg per day.

8. Use according to claim 5 wherein the rapamycin is administered in a dose range from 0.01 to 0.5 mg/kg per day.

9. Use according to any one of claims 1 to 4 wherein the rapamycin is administered orally.

10. Useaccording to claim 9 wherein the rapamycin is administered in a dose range from 0.005 - 75 mg/kg per day.

11. Use according to claim 9 wherein the rapamycin is administered in a dose range from 0.01 - 50 mg/kg per day.

12. Use according to claim 9 wherein the rapamycin is administered in a dose range from 0.05 - 10 mg/kg per day.
